(19) Europäisches Patentamt
European Patent Office
Office européen des brevets

(11) Veröffentlichungsnummer: **0 162 394**
**B1**

(12) # EUROPÄISCHE PATENTSCHRIFT

(45) Veröffentlichungstag der Patentschrift: **27.06.90**

(21) Anmeldenummer: **85105838.8**

(22) Anmeldetag: **13.05.85**

(51) Int. Cl.[5]: **C 07 D 501/46,** A 61 K 31/545
// C07D501/18, C07D501:24,
C07D501:42

(54) **Neue Cephalosporine und Verfahren zu ihrer Herstellung.**

(30) Priorität: **22.05.84 DE 3419013**

(43) Veröffentlichungstag der Anmeldung:
**27.11.85 Patentblatt 85/48**

(45) Bekanntmachung des Hinweises auf die Patenterteilung:
**27.06.90 Patentblatt 90/26**

(84) Benannte Vertragsstaaten:
**AT BE CH DE FR GB IT LI NL SE**

(56) Entgegenhaltungen:
**EP-A-0 121 244**
**EP-A-0 137 440**
**FR-A-2 366 297**
**GB-A-2 116 180**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Angerbauer, Rolf, Dr.**
**Sterntalerweg 29**
**D-5600 Wuppertal 1 (DE)**
Erfinder: **Boberg, Michael, Dr.**
**Untere Bergerheide 47**
**D-5600 Wuppertal 1 (DE)**
Erfinder: **Metzger, Karl Georg, Dr.**
**Pahlkestrasse 75**
**D-5600 Wuppertal 1 (DE)**
Erfinder: **Zeiler, Hans-Joachim, Dr.**
**Elsbeekerstrasse 46**
**D-5690 Velbert 15 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 162 394 B1

**Beschreibung**

Die Erfindung betrifft neue Cephalosporine, ihre Verwendung als Arzneimittel, insbesondere in der antibakteriellen Therapie sowie Verfahren zu ihrer Herstellung und Zwischenprodukte für die Herstellung der Cephalosporine.

Cephalosporine, die als Acylseitenkette einen 2-(2-Aminothiazol-4-yl)-2-alkoxyiminoessigsäurerest und in 3-Stellung einen Pyridiniummethyl-Rest tragen, sind aus EP—0-064 740 bekannt.

Gemäß Art. 54(3) EPÜ gehören auch die EP—A—O 121 244 und die EP—A—O 137 440 zum Stand der Technik.

Außerdem in der FR—PS 2 366 297 Cephalosphorine beschrieben, die sich jedoch in der Seitenkette strukturell sehr stark von den erfindungsgemäßen Verbindungen unterscheiden.

Durch die vorliegende Erfindung werden Cephalosporine der allgemeinen Formel I und deren pharmazeutisch verträgliche Salze zur Verfügung gestellt,

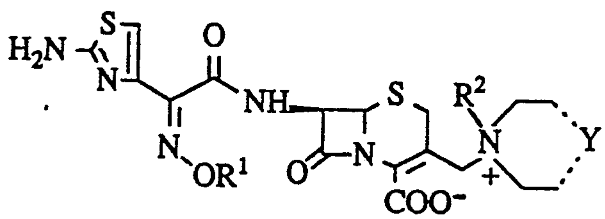

(I)

in der

$R^1$ einen $C_1$—$C_3$-Alkylrest darstellt und

$R^2$ für Propyl, Isopropyl, Cyclopropyl, Chlorethyl, Methoxymethyl, Methoxyethyl, Vinyl, Carboxyethyl oder Aminoethyl steht, und

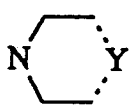 für einen Rest der Formel

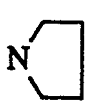 , 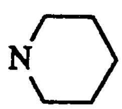 ; 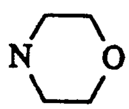 , 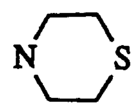 , 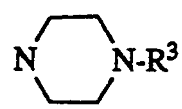

steht, worin $R^3$ die oben für $R^2$ genannte Bedeutung hat.

Bevorzugt steht $R^1$ für einen Methylrest.

Die Verbindungen der allgemeinen Formel I können dadurch erhalten werden, daß Verbindungen der allgemeinen Formel II

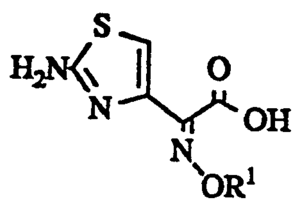

(II)

worin

$R^1$ die oben genannte Bedeutung hat,

in denen die Amingruppe geschützt oder ungeschützt vorliegen kann, nach Aktivierung der Carboxylgruppe durch Überführung in ein gemischtes Anhydrid, beispielsweise mit Chloroameisensäureethylester oder Methansulfonsäurechlorid, nach Überführung in das Säurehalogenid oder nach Überführung in einen aktivierten Ester mit beispielsweise N-Hydroxybenztriazol und Dicyclohexylcarbodiimid, mit Verbindungen der allgemeinen Formel III,

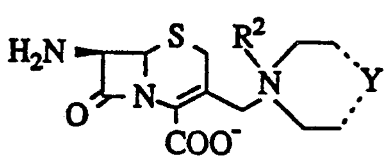

(III)

worin $R^2$ und

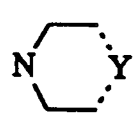

die oben genannte Bedeutung besitzen, zur Umsetzung gebracht werden, dann gegebenenfalls Schutzgruppen abgespalten werden und die gewünschten Salze oder aus Salzen die freien Säuren hergestellt werden.

Für die Kupplung von Carbonsäuren (II) an β-Lactame der Formel (III) kann eine große Zahl von aus der Cephalosporin- bzw. Penicillinchemie bekannten Methoden verwendet werden. Es hat sich als vorteilhaft erwiesen, die Carbonsäuren der allgemeinen Formel (II) ohne Amin-Schutzgruppe zu aktivieren und dann mit den ß-Lactamen der Formel (III), die als Salze mit einem Amin in Lösung gebracht wurden, zu kuppeln. Besonders vorteilhaft ist die Aktivierung mit Sulfonsäurederivaten der Formel (V) zu Anhydriden der Formel (IV)

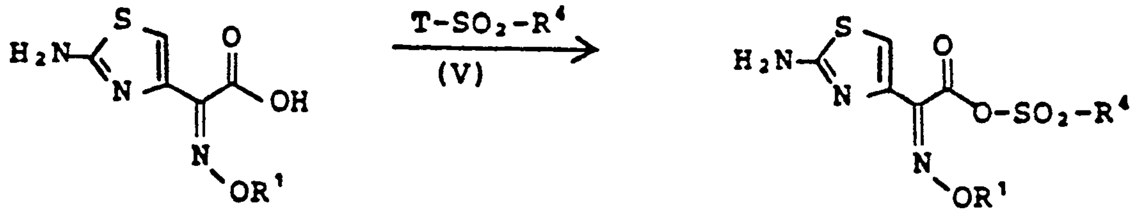


(II) (IV)

worin

T einem Rest $R^4—SO_2—O—$ oder Halogen darstellt und

$R_4$ einen Alkylrest mit 1—10 C-Atomen, der gegebenenfalls durch Fluor, Chlor, CN, Phenyl, Alkyloxycarbonyl, Alkyloxy oder Alkyl substituiert sein kann, wobei letztere Alkylreste 1—4 C-Atome tragen können, oder einen Phenylrest, der gegebenenfalls durch Fluor, Chlor, Brom, CN, Alkyl, Alkyloxy, Alkylthio, Alkyloxycarbonyl — wobei letztere Alkylgruppen 1—4 C-Atome tragen können — Nitro, Trifluormethyl und Phenyl substituiert sein kann, bedeutet.

Wenn $R^4$ substituiert ist, sind vorzugsweise 1—3 Substituenten, vorzugsweise die genannten vorhanden.

Ganz besonders bevorzugt stellt $R^4$ einen Methyl- oder p-Tolylrest dar.

Die gemischten Anhydride der Formel (IV) werden hergestellt, indem man die Carbonsäuren der Formel (II) und 1—1, 4 Äquivalente eines Amins in einem Lösungsmittel löst und mit 1 bis 1,2 Äquivalenten eines Sulfonsäurederivates der Formel (V) reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformid.

Als Amin eignen sich tertiäre Amine wie z.B. Triethylamin oder Tributylamin, aber auch sterisch gehinderte sekundäre Amine wie z.B. Diisopropylamin.

Die Umsetzungen können bei Temperaturen zwischen −80°C und Raumtemperatur durchgeführt werden, wobei tiefe Temperaturen eine Isomerisierung der Substituenten an der Doppelbindung vermeiden. Vorteilhaft wird die Aktivierung mit $Cl—SO_2CH_3$, in Dimethylformamid bei −40 bis −60°C innerhalb von 0,2 bis 24 Stunden, vorzugsweise 0,5 bis 5 Stunden, durchgeführt.

Zum Lösen der Verbindungen der Formel (III) können die bei der Herstellung der Verbindungen der Formel (IV) genannten Lösungsmittel oder Wasser sowie als Base die dort genannten Amine verwendet werden.

Besonders vorteilhaft ist auch eine Aktivierung der Carbonsäuren der allgemeinen Formel (II) durch überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel (IV) eignen.

Die Umsetzungen können bei Temperaturen zwischen −30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit einer Verbindung der Formel (III) in Form einer Lösung ihres Aminsalzes innerhalb von 2 bis 24 Stunden umgesetzt. Zum Lösen der Verbindungen der Formel (III) können die bei der Herstellung der Verbindungen der Formel (IV) genannten Lösungsmittel sowie als Base die dort genannten Amine verwendet werden.

Die Verbindungen der Formel (III) erhält man, indem man aus Verbindungen der Formel (VI) die Aminschhutzgruppe $R^5$ abspaltet.

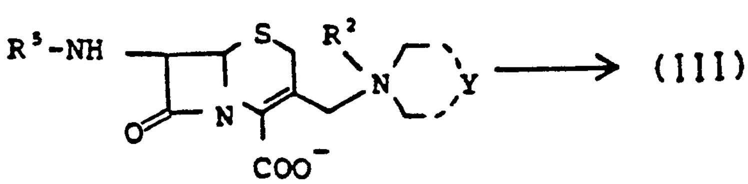


(VI)

Dabei kann $R^5$ entweder eine säurelabile Schutzgruppe wie die t-Butyloxycarbonylgruppe oder vorteilhaft eine enzymatisch abspaltbare Schutzgruppe sein. Bevorzugte enzymatisch abspaltbare Schutzgruppen sind Phenacetyl oder 2-Thienylacetyl.

Die enzymatische Abspaltung erfolgt bei Raumtemperatur in Wasser oder einem Gemisch aus Wasser und einem polaren organischen Lösungsmittel, wie z.B. Acetonitril oder Tetrahydrofuran, mit immobilisierter Penicillin G Acylase bei pH 7—8, bevorzugt bei pH 7,5—7.8.

Während der enzymatischen Spaltung wird der pH-Wert durch Zugabe einer Base wie Lithiumhydroxid, Natriumhydroxid, Kaliumhydroxid oder eines tert. Amins wie z.B. Triethylamin, Tripropylamin, Tributylamin oder Pyridin konstant gehalten.

Die Verbindungen der Formel (VI) lassen sich aus Estern der Formel (VII) über Zwischenverbindungen der Formel (VIII) herstellen.

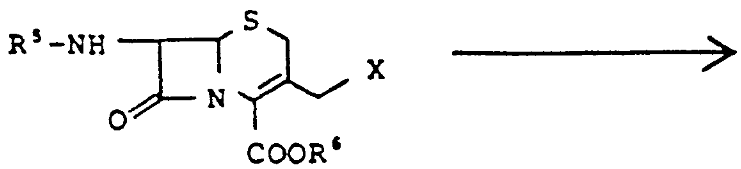
(VII)

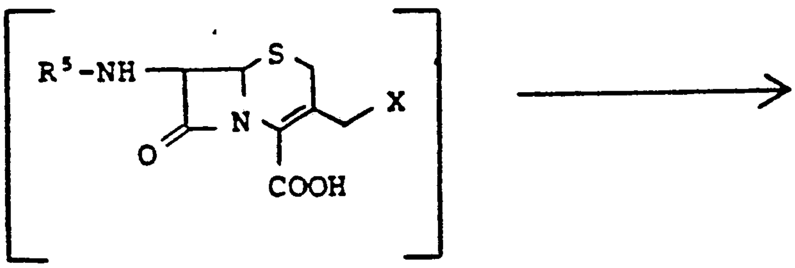
(VI) (VIII)

In den Estern der Formel (VII) stellt X eine Fluchtgruppe, wie Mesylat, Tosylat, Brosylat, Triflat, Nonaflat, Jodid, Bromid, Chlorid, und $R^6$ eine in der Cephalosporinchemie übliche Säureschutzgruppe, bevorzugt eine sauer abspaltbare Schutzgruppe, wie z.B. Benzhydryl, 4-Methoxydiphenylmethyl, t-Butyl, dar.

Die Verbindungen der Formel (VII) werden durch Abspaltung der Säureschutzgruppe $R^6$ in die reaktiven freien Säuren der Formel (VIII) überführt. Bei den bevorzugten säurelabilen Schutzgruppen $R^6$ wird die Schutzgruppe in einem organischen Lösungsmittel abgespalten. Bevorzugt ist die Abspaltung der Benzhydrylschutzgruppe in Methylenchlorid mit Trifluoressigsäure möglicherweise unter Zusatz eines Alkoxybenzols, bevorzugt Methoxybenzol. Die Abspaltung erfolgt bei −20°C bis +30°C, bevorzugt bei 0°C innerhalb von 5 Minuten bis einer Stunde, bevorzugt innerhalb von 20 Minuten.

Die Säure der Formel (VIII) kann nach Abspaltung der Schutzgruppe isoliert werden. Vorteilhaft wird jedoch nicht isoliert, sondern direkt und ohne Reinigung zu Verbindungen der Formel (VI) umgesetzt. Dazu wird die bei der Reaktion (VII) → (VIII) entstehende Lösung von (VIII) schonend im Vakuum eingeengt. Die zurückbleibende rohe Säure wird in einem organischen Lösungsmittel, bevorzugt in Tetrahydrofuran, aufgenommen und mit 2—50 Äquivalenten, bevorzugt mit 5—20 Äquivalenten, eines tert. Amins der Formel

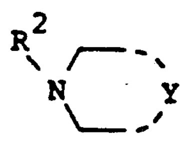

wobei $R^2$ und

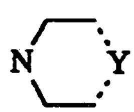

die vorstehend genannte Bedeutung haben, zu Verbindungen der Formel (VI) umgesetzt.

Die Umsetzung wird bei Temperaturen zwischen −20°C und 40°C, bevorzugt bei 25°C, innerhalb von 10 Minuten bis zwei Stunden, bevorzugt innerhalb von 30 Minuten durchgeführt. Das Produkt kann nach Beendigung der Reaktion durch Zugabe von Diethylether ausgefällt werden. Das so erhaltene Rohprodukt kann an einem Harz wie Diaion HP 20 oder XAD 7 gereinigt werden. Es ist auch vorteilhaft möglich, das Rohprodukt direkt zu Verbindungen der Formel (III) weiter umzusetzen.

Die Verbindungen der Formel (VI) lassen sich alternativ auch aus Säuren der Formel (IX) herstellen,

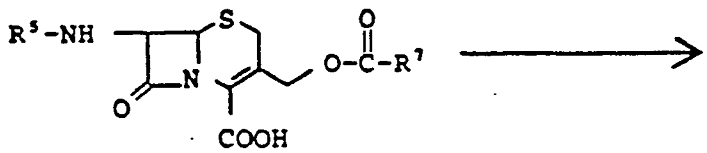

(IX)

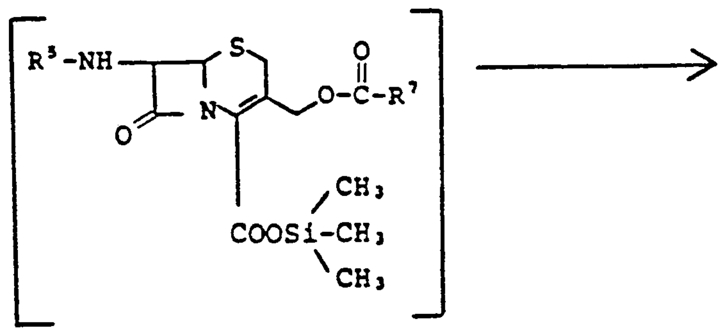

(X)

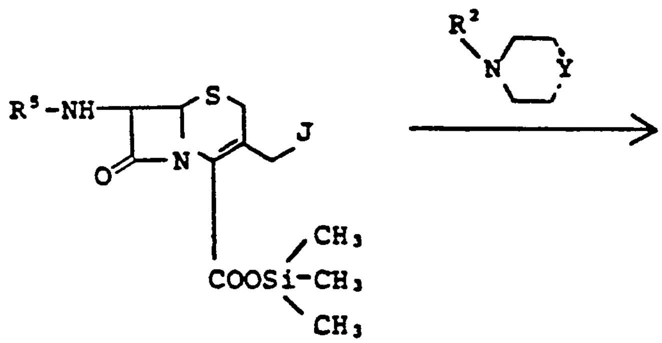

(VI)

(XI)

in denen $R^5$ die vorstehend genannte Bedeutung hat und $R^7$ einen gegenbenenfalls substituierten Alkyl- oder Arylrest darstellt, wie Methyl, Ethyl, Propyl, Chlormethyl, Dichlormethyl, Trichlormethyl, Trifluormethyl, Phenyl. Ganz besonders bevorzugt stellt $R^7$ eine Methylgruppe dar.

Die Ausgangsverbindungen der Formel (IX) werden in einem geeigneten organischen Lösungsmittel suspendiert und durch Silylierung zum Silylester XI in Lösung gebracht. Besonders geeignete organische Lösungsmittel sind Chloroform, Methylenchlorid, Dichlorethan. Die Silylierung wird mit einem üblichen Silylierungsmittel wie Trimethylchlorsilan (TMCS), Hexamethyldisilazan (HMDS), N,O-Bis-(trimethylsilyl)acetamid (BSA) N,O-Bis(trimethylsilyl)-trifluoracetamid (BSTFA), N-Methyl-N-trimethylsilylacetamid (MSA), N-Methyl-N-trimethylsilyltrifluoracetamid (MSTFA), 1,3-Bis(trimethylsilyl)harnstoff oder Trimethylsilyltrifluoromethansulfonat durchgeführt. Dabei können auch mehrere Silylierungsmittel im Gemisch eingesetzt werden.

Die Silylierung erfolgt bei −30°C bis +70°C, bevorzugt bei −10°C bis +10°C, innerhalb von 5 Minuten bis 30 Minuten. Vorteilhaft wird ein bis zu zehnfacher Überschuß des Silylierungsmittels eingesetzt, bevorzugt ein zweibis fünffacher Überschuß.

Die so erhaltene Lösung des Trimethylsilylesters der Formel (X) wird bei −40°C bis +30°C, bevorzugt bei −10°C bis +10°C, mit ein bis zehn Äquivalenten, bevorzugt mit drei bis vier Äquivalenten eines Trialkylsilyljodids, besonders bevorzugt Trimethylsilyljodid, innerhalb von 15 Minuten bis 2 Stunden, bevorzugt innerhalb von 30 Minuten bis 1 Stunde, zu Verbindungen der Formel (XI) umgesetzt.

Die Verbindungen der Formel XI werden vorteilhaft nicht isoliert, sondern direkt ohne Reinigung mit Aminen

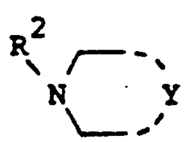

zu den Verbindungen der Formel VI umgesetzt.

Die Verbindungen der allgemeinen Formel I können alternativ auch dadurch hergestellt werden, daß Verbindungen der Formel (XII),

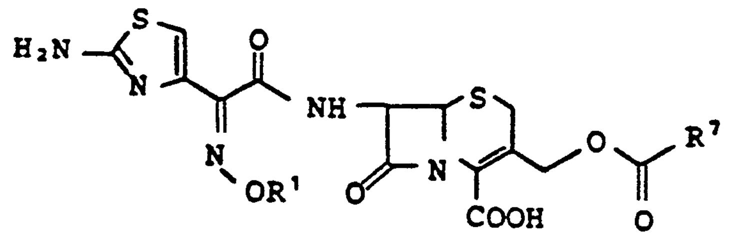

XII

in denen $R^1$ und $R^7$ die oben genannte Bedeutung haben, analog wie vorher für die Umsetzung von Verbindungen der Formel X zu Verbindungen der Formel VI beschrieben, direkt ohne Isolierung der Zwischenstufen nach Silylierung und Überführung ins Jodid mit Aminen

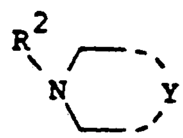

zu Verbindungen der Formel I umgesetzt werden.

Die erfindungsgemäßen Verbindungen weisen eine starke und breite antimikrobielle Wirksamkeit besonders gegen gramnegative und grampositive Bakterien auf, Diese Eigenschaften ermöglichen ihre Verwendung als chemotherapeutische Wirkstoff in der Medizin.

Besonders wirksam sind die erfindungsgemäßen Verbindungen gegen Bakterien und bakterieähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken. z.B. Staphylococcus aureas, Staph. epidermidis, Staph. aerogenes und Graffkya tetragena (Staph. = Staphylococcus);

Lactobacteriaceae wie Streptokokken, z.B. Streptococcus pyogenes, α-bzw. β-hämolysierende Streptokokken, nicht (δ-)-hämolysierende Streptokokken, Str. viridans, Str. faecalis (Enterokokken) und Dipolococcus pneumoniae (Pneumokokken) (Str. = Streptococcus);

Enterobacteriaceae, wie Escherichiae-Bakterien der Coli-Gruppe: Escherichia-Bakterien, z.B. Escherichia coli, Enterobacter-Bakterien, z.B. E. aerogenes, E. cloacae, Klebsiella-Bakterien, z.B. K. pneumoniae, Serratia, z.B. Serratia marcescens (E. = Enterbacter) (K. = Klebsiella), Proteae-Bakterien der Proteus-Gruppe: z.B. Proteus vulgaris, Pr. morganii, Pr. rettgeri, Pr. mirabilis (Pr. = Proteus);

Pseudomonadaceae, wie Pseudomonas-Bakterien, z.B. Pseudomonas aeruginosa (Ps. = Pseudomonas);

Bacteroidaceae, wie Bacteroides-Bakterien, z.B. Bacteroides fragilis (B = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können, seien beispielsweise genannt:

Erkrankungen der Atemwege und des Rachenraumes;

Otitis, Pharyngitis; Pneumonie; Peritonitis, Pyelonephritis, Cystitis, Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewßöhnlich einer ganzen, einer halben oder einem Drittel oder einem Viertel einer Tagesdosis entspricht.

Unter nichttoxischen inerten pharmazeutische geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den die Wirkstoffe neben den üblichen Trägerstoffen enthalten wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calcium-carbonat und Natriumcarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmitteln enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffe die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Zur parenteralen Applikation können die Lösungen auch in steriler und blutisotonischer Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoff enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Human- als auch in der Verterinärmedizin als vorteilhaft erwiesen, den oder die erfindungsgemäßen Wirkstoffe in Gesamtmengen von etwa 1 bis etwa 1 000, vorzugsweise 1 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 250, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankungen, der Art der Zubereitung und der Applikation des Arzneimittels sowie dem Zeitraum bzw. Intervall des Arzneimittels sowie dem Zeitraum bzw. Intervall innerhalb welchem die Verabreichung erfolgt. So kann es in einigen Fällen ausreiched sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums mit einem anderen β-Lactamantibiotikum oder auch mit Aminoglykosidantibiotika, wie z.B. Gentamicin, Sisomicin, Kanamicin, Amikacin oder Tobramicin kombiniert werden.

Die erfindungsgemäßen Wirkstoffe können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der Wirkstoffe ist hierbei weitgehend unabhängig von der Art und dem Geschlecht der Tiere. Besonders wertvoll erweisen sich die Wirkstoffe bei der Aufzucht und Haltung von Jung- und Masttieren. Als Tiere, bei denen die Wirkstoffe zur Förderung und Beschleunigung des Wachstums und zur Verbesserung der Futterverwertung eingesetzt werden können, seien beispielsweise folgende Nutz- und Ziertiere genannt:

Warmblüter wie Rinder, Schweine, Pferde, Schafe, Ziegen, Katzen, Hunde, Kaninchen; Pelztiere, z.B., Nerze und Chinchilla; Geflügel, z.B. Hühner, Gänse, Enten, Truthähne, Tauben Papageien und Kanarienvögel; und Kaltblüter wie Fische, z.B. Karpfen, und Reptilien, z.B. Schlangen.

Die Mengen der Wirkstoffe, die den Tieren zur Erreichung des gewünsten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die zu verabreichende Menge des Wirkstoffs sowie die entsprechende Dauer der Verabreichung hängen insbesondere von der Art, dem Geschlecht, dem Gesundheitszustand und der Art der Haltung und

Fütterung der Tiere ab und sind durch jeden Fachmann leicht zu ermitteln.

Die Wirkstoffe werden den Tieren nach den üblichen Methoden verabreicht. Die Art der Verbreichtung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen. Aus Zweckmäßigkeitsgründen ist in den meisten Fällen eine orale Verabreichung, insbesondere im Rhytmus der Nahrungs- und/oder Getränkeaufnahme der Tiere, vorzuziehen. Unter Nahrung in Sinne der vorliegenden Erfindung werden sowohl feste als auch flüssige Nahrung und auch Getränke und Wasser verstanden.

Die Wirkstoffe können als reine Stoffe oder in formulierter Form, also in Mischung mit nichttoxischen inerten Trägerstoffen beliebiger Art, z.B. mit Trägerstoffen und im Formulierungen, wie sie bei nutritiven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitamnen, Einweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Wirkstoff der Gesamtmenge oder nur Teilen des Futters und/oder Trinkwassers zugegeben wird.

Die Wirkstoffe werden nach üblichen Methoden durch einfaches Mischen als reine Stoffmischung, vorzugsweise in fein verteilter Form oder in formulierter Form in Mischung mit eßbaren nichttoxischen Trägerstoffen, gegebenenfalls in Form eines Praemix oder eines Futterkonzentrates, dem Futter und/oder Trinkwasser beigefügt ist, formaliert.

Das Futter und/oder Trinkwasser kann beispielsweise die Wirkstoffe in einer Gewichtskonzentration von etwa 0,01 bis 50, insbesondere 0,1 bis 10 ppm enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kaln und Kochsalz.

Futterkonzentrate enthalten die Wirkstoffe neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe gegebenenfalls auch durch ihre Oberfläche bedeckende geeignete Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält.

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischem 1 kg Futter.

In einem kg Futtermischung sind enthaltend: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7\ H_2O$, 100 mg $FeSO_4 \times 7\ H_2O$ und 20 mg $CuSO_4 \times 5\ H_2O$.

Der Wirkstoff-Praemix enthält die Wirkstoffe in der gewünschten Menge, z.B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält.

630 g Futtergetreideschrot (zusammensetzung aus 200 g Mais-, 150 g Gerste-, 150 g Hafer- und 130 g Weizenschrot); 80 g Fischmehl, 60 g Sojaschrot, 60 g Tapiokamehl, 38 g Bierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zukkerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

## Beispiel 1

3-Chloromethyl-7β-phenylacetamido-3-cephem-4-carbonsäurebenzhydrylester

Zu einer Lösung von 103 g (0,2 Mol) 3-Hydroxymethyl-7β-phenylacetamido-3-cephen-4-carbonsäurebenzhydrylester (hergestellt z.B. nach Helv. Chim. Acta *57,* 2044 (1974)) in 3,5 l absol. Tetrahydrofuran gibt man unter Eiskühlung 24 ml (0,3 Mol) Pyridin, 400 µl Dimethylformamid und 21,6 ml (0,3 Mol) Thionylchlorid. Nach 10 Minuten wird am Rotationsverdampfer eingeengt, mit 2 l Essigester aufgenommen und zweimal mit Natriumhydrogencarbonatlösung und einmal mit Wasser ausgeschüttelt. Die organische Phase wird mit je 50 g Kieselgur und Aktivkohle ausgerührt und über eine mit Kieselgel belegte Fritte abgesaugt. Anschließend wird über Magnesiumsulfat getrocknet, eingeengt, in 200 ml Methylenchlorid aufgenommen und mit Petrolether ausgefällt.

Ausbeute: 76 g

$^1$H-NMR ($DCCl_3$)

δ (ppm) = 7,20—7.50 (15H, m, arom.); 6.96 (1H, s, $CH\phi_2$); 6,30 (1H, d, J=9Hz, NH), 5,86 (1H, dd, J=9 Hz, J=5 Hz, H—7); 4,95 (1H, d, J=5 Hz, H—6); 4,36 (2H, bs, $DH_2$—Cl); 3,66 (1H, d, J=15 Hz, φ—$CH_2$-); 3,58 (1H, d, J=15 Hz, φ—$CH_2$-); 3,56 (1H, d, J=18 Hz, H—2); 3.40 (1H, d, J=18 Hz, H—2).

Beispiel 2 (nicht erfindungsgemäß)

7-Amino-3-(1-ethyl-1-pyrrolidinium)methyl-3-cephem-4-carboxylat

10 g (18,8 mmol) 3-Chloromethyl-7β-phenylacetamido-3-cephem-4-carbonsäurebenzhydrylester werden bei 0°C in 112 ml absol. Methylenchlorid gelöst. Nach Zugabe von 56 ml Anisol und 56 ml Trifluoressigsäure wird 25 Minuten bei 0°C gerührt. In Vakuum wird eingeengt, 100 ml Benzol werden zugegeben und der Ansatz wird 1 h im Hochvakuum eingeengt. Der Rückstand wird in 100 ml absol. Tetrahydrofuran gelöst und 18,6 g (188 mmol) N-Ethylpyrrolidin werden zugegeben. Die Lösung wird 30 Minuten bei Raumtemperatur gerührt. 100 ml Ether werden zugegeben. Der entstehende Niederschlag wird abgesaugt, mit 500 ml Ether gewaschen und durch Zugabe von $NaHCO_3$ in 50 ml Wasser gelöst. Anschließend werden 4 g immobilisierter Penicillin-G-Acylase zugegeben und der pH-Wert wird durch Zugabe von 4N-Triethylamin in Ethanol konstant bei 7.8 gehalten. Nach Beendigung der enzymatischen Spaltung wird von der Acylase abfiltriert und das Filtrat mit konz. Salzsäure auf pH 2 gestellt. Vom entstehenden Niederschlag wird über Kieselgel abgesaugt und das Filtrat wird zu 2 l Aceton getropft. Das gewünschte Produkt kristallisiert als Hydrochlorid aus und wird abgesaugt und getrocknet.

Ausbeute: 1.76 g (xHClx$H_2O$, 25.6%).

NMR ($D_2O$)

δ (ppm) = 5.31 (1H, d, J=5Hz, H-7-Lactam); 5.12 (1H, d, J=5Hz), H-6-Lactam); 4.62 (1H, d, J=14Hz, $CH_2$-Pyrrol.); 3.88 (1H, d, J=14Hz, $CH_2$-Pyrrol.); 3.86 (1H, d, J=18Hz, S—$CH_2$); 3.58 (1H, d, J=18Hz, S—$CH_2$); 3.42 (4H, m, Pyrrol.); 3.24 (2H, q, J=7Hz,

$$-CH_2-\overset{|}{\underset{|}{N^+}}-);$$

2.06 (4H, m, Pyrrol.); 1.24 (3H, t, J=7Hz, $CH_3$).

Beispiel 3 (nicht erfindungsgemäß)

7β-[(Z)-2-(2-Aminothiazol-4-yl)-2-methoxyiminoacetamido]-3-(1-ethyl-1-pyrrolidinium)methyl-3-cephem-4-carboxylat

353 mg (1.76 mmol) (Z)-2-(2-Aminothiazol-4-yl)-2-methoxyiminoessigsäure werden unter Stickstoff bei Raumtemperatur in 2,7 ml absol. Dimethylformamid gelöst. Nach Zugabe von 113 µl N-Ethyldiisopropylamin, 123 µl Tripropylamin und 152 µl Tributylamin wird auf −50°C gekühlt. 145 µl Methansulfonsäurechlorid werden zugegeben und die Lösung wird 30 Min. bei −50°C gerührt. Anschließend wird diese Lösung schnell zu einer auf 0°C gekühlten Lösung von 470 mg (1.35 mmol) 7-Amino-3-(1-ethyl-1-pyrrolidinium)-methyl-3-cephem-4-carboxylat (x HCl) in 0,85 ml Wasser und 0,7 ml Triethylamin gegeben. Nach 5 Min. wird die Reaktionslösung auf 150 ml Aceton gegeben. Der entstehende Niederschlag wird abgesaugt, getrocknet und über Adsorberharz HP 20 chromatographiert (Elutionsmittel: Acetonitril/Wasser 5/95).

Ausbeute: 400 mg (59%).

$^1$H-NMR ($D_6$-DMSO)

δ (ppm) = 9.63 (1H, d, J=9Hz, NH); 7.28 (2H, bs, $NH_2$); 6.78 (1H, s, Thiazol); 5.69 (1H, dd, J=9Hz, H=5Hz, H-7-Lactam); 5.15 (1H, d, J=5Hz, H-6-Lactam); 5.08 (1H, d, J=14Hz, $CH_2$-Pyrrol.); 3.88 (1H, s, $OCH_3$); 3.85 (1H, d, J=14Hz, $CH_2$-Pyrrol.); 3.81 (1H, d, J=18Hz, S—$CH_2$); 3.20—3.50 (7H, m, S—$CH_2$, —$CH_2$—N—, Pyrrol.); 2.04 (4H, m, Pyrrol.); 1.28 (3H, t, J=7Hz, $CH_3$).

Beispiel 4 (nicht erfindungsgemäß)

3-[(1-(2-Hydroxyethyl)-1-pyrrolidinium]methyl-7β-phenylacetamido-3-cephem-4-carboxylat

Unter Stickstoff werden 4.68 g (12 mmol) 3-Acetoxymethyl-7β-phenylacetamido-3-cephem-4-carbonsäure bei Raumtemperatur in 48 ml absol. Methylenchlorid aufgeschlämmt und durch Zugabe von 7,6 ml (36 mmol) N-Methyl-N-trimethylsilyltrifluoracetamid (MSTFA) in Lösung gebracht. Nach Abkühlen auf 0°C werden 7 ml (48 mmol) Trimethylsilyljodid zugegeben und die Reaktionslösung wird 1 h bei 0°C gerührt. Nach Zugabe von 7,6 absol. Tetrahydrofuran wird weitere 15 Min. bei 0°C gerührt. Anschließend werden 14,4 ml (120 mmol) N-(2-Hydroxyethyl)pyrrolidin zugegeben, und die Lösung wird 30 Min. nachgerührt. Dann werden 2,4 ml Wasser zugegeben und nach weiteren 5 Min. wird auf 200 ml Ether gegossen. Der Ether wird vom öligen Rückstand abdekantiert, der Rückstand nochmals mit Ether verrührt und nach erneutem Dekantieren in Wasser aufgenommen und über Adsorberharz HP 20

chromatographiert (Elutionsmittel: Acetonitril/Wasser 5/95).

Ausbeute: 3.6 g (68%).

$^1$H-NMR ($D_6$-DMSO)

δ (ppm) = 9.13 (1H, d, J=9Hz, NH); 7.28 (5H, m, arom.); 5.55 (1H, dd, J=9Hz, J=5Hz, H-7-Lactam); 5.06 (1H, d, J=5Hz, H-6-Lactam); 5.04 (1H, d, J=14Hz, $CH_2$-Pyrrol.); 3.95 (1H, d, J=14Hz, $CH_2$-Pyrrol.); 3.33—3.85 (12H, m); 2.04 (4H, m, Pyrrol.).

Beispiel 5 (nicht erfindungsgemäß)

7-Amino-3-[1-(2-hydroxyethyl)-1-pyrrolidinium]methyl-3-cephem-4-carboxylat

Zu einer Lösung von 3,5 g (7.8 mmol) 3-[1-(2-Hydroxyethyl-1-pyrrolidinium]methyl-7β-phenylacetamido-3-cephem-4-carboxylat in 100 ml Wasser werden 4 g immobilisierter Penicillin-G-Acylase gegeben und der pH-Wert wird durch Zugabe von 4N Triethylamin in Ethanol konstant bei 7.8 gehalten. Nach Beendigung der enzymastischen Spaltung wird von der Acylase abfiltriert und das Filtrat wird mit konz. Salzsäure auf pH 2 gestellt. Vom entstehenden Niederschlag wird über Kieselgel abgesaugt und das Filtrat wird zu 2 l Aceton getropft. Des gewünschte Produkt kristallisiert als Hydrochlorid aus und wird abgesaugt und getrocknet.

Ausbeute: 1.9 g (xHClx$H_2O$, 64%).

$^1$H-NMR ($D_6$-DMSO)

δ (ppm) = 5.33 (1H, d, J=5Hz, H-7-Lactam); 5.13 (1H, d, J=5Hz, H-6-Lactam); 4.70 (1H, d, J=14Hz, $CH_2$-Pyrrol.); 3.93 (2H, m, $CH_3$—OH); 3.87 (1H, d, J=18Hz, S—$CH_2$); 3.30—3.70 (7H, m); 2.11 (4H, m, Pyrrol.).

Beispiel 6

7-Amino-3-(1-propyl-1-pyrrolidinium)methyl-3-cephem-4-carboxylat

Die Herstellung erfolgt analog Beispiel 1 aus 3-Chloromethyl-7β-phenylacetamido-3-cephem-4-carbonsäurebenzhydrylester und N-Propylpyrrolidin

$^1$H-NMR ($D_2O$)

δ (ppm) = 5.33 (1H, d, J=5Hz, H-7-Lactam); 5.15 (1H, d, J=5Hz, H-6-Lactam); 4.63 (1H, d, J=13Hz, $CH_2$-Pyrrol.); 3.96 (1H, d, J=13Hz, $CH_2$-Pyrrol.); 3,88 (1H, d, J=18Hz, S—$CH_2$); 3,55 (1H, d, J=18Hz, S—$CH_2$); 3,45 (4H, m, Pyrrol.); 3.12 (2H, m,

$$-CH_2-{}^{+}\overset{|}{\underset{|}{N}}-);$$

2,10 (4H, m, Pyrrol.); 1,66 (2H, m, —$CH_2$); 0,85 (3H, m, $CH_3$).

Beispiel 7

7β[(Z)-2-(2-Aminothiazol-4-yl)-2-methoxyiminoacetamido]-3-(1-propyl-1-pyrrolidinium)methyl)-3-cephem-4-carboxylat

Die Herstellung erfolgt analog Beispiel 3 aus (Z)-2-(2-Aminothiazol-4-yl)-2-methoxyiminoessigsäure und 7-Amino-3-(1-propyl-1-pyrrilidinium)methyl-3-cephem-4-carboxylat

$^1$H-NMR (DMSO-$d_6$)

δ (ppm) = 9.58 (1H, d, J=9Hz, NH); 7.24 (2H, bs, $NH_2$); 6.75 (1H, s, Thiazol); 5,67 (1H, dd, J=9Hz, J=5Hz, H-7-Lactam); 5,13 (1H, d, J=5Hz, H-6-Lactam); 5,08 (1H, d, J=13Hz, $CH_2$-Pyrrol.); 3.86 (3H, s, $OCH_3$); 3,80 (2H, m, $CH_2$-Pyrrol., S—$CH_2$); 3,45 (5H, m, Pyrrol., S—$CH_2$); 3,13 (2H, m,

$$-CH_2-{}^{+}\overset{|}{\underset{|}{N}}-),$$

2.03 (4H, m, Pyrrol.); 1,74 (2H, n, —$CH_2$—); 0,90 (3H, t, J=7Hz, $CH_3$).

Beispiel 8

7-Amino-3-(1-isopropyl-1-pyrrolidinium)methyl-3-cephem-4-carboxylat

Die Herstellung erfolgt analog Beispiel 2 aus 3-Chloromethyl-7β-phenylacetamido-3-cephem-4-carbonsäurebenzhydrylester und N-Isopropylpyrrolidin

$^1$H-NMR ($D_2O$)

δ (ppm) = 5,35 (1H, d, J=5Hz, H-7-Lactam; 5,13 (1H, d, J=5Hz, H-6-Lactam); 4,02 (1H, d, J=13Hz, $Ch_2$-Pyrrol.); 3.93 (1H, d, J=18Hz, S—$CH_2$); 3,40—3,80 (6H, m, S—$CH_2$, Pyrrol.,

$$>CH-{}^{+}\overset{|}{\underset{|}{N}}-;$$

2,10 (4H, m, Pyrrol.); 1,43 (6H, m, Isoprop.).

Beispiel 9

7β-[(Z)-2-(2-Aminothiazol-4-yl)-2-methoxyiminoacetamido]3-(1-isopropyl-1-pyrrolidinium)methyl-3-cephem-4-carboxylat

Die Herstellung erfolgt analog Beispiel 3 aus (Z)-2-(2-Aminothiazol-4-yl)-2-methoxyiminoessigsäure und 7-Amino-3-(1-isopropyl-1-pyrrolidinium)methyl-3-cephem-4-carboxylat

$^1$H-NMR (DMSO-$d_6$)

δ (ppm) = 9.61 (1H, d, J=9Hz, NH); 7.28 (2H, s, $NH_2$); 6,77 (1H, s, Thiazol); 5.67 (1H, dd, J=9Hz, J=5Hz, H-7-Lactam); 5,17 (1H, d, J=5Hz, H-6-Lactam); 4,96 (1H, d, J=13Hz, $CH_2$-Pyrrol.); 3,92 (1H, d, J=13Hz, $CH_2$-Pyrrol.); 3,87 (3H, s, $OCH_3$); 3,83 (1H, d, J=18Hz, S—$CH_2$); 3.40—3,70 (6H, m,

>CH—$^+$N—;

Pyrrol., S—$CH_2$); 1,98 (4H, m, Pyrrol.); 1,35 (6H, m, Isoprop.).

Beispiel 10

7-Amino-3-(1-butyl-1-pyrrolidimium)methyl-3-cephem-4-carboxylat

Die Herstellung erfolgt analog Beispiel 2 aus 3-Chloromemethyl-7β-phenylacetamido-3-cephem-4-carbonsäurebenzhydrylester und N-Butyl-pyrrolidin

$^1$H-NMR ($D_2O$)

δ (ppm) = 5.24 (1H, d, J=5Hz, H-7-Lactam); 5.04 (1H, d, J=5Hz, H-6-Lactam); 4,58 (1H, d, J=13Hz, $CH_2$-Pyrrol.); 3,85 (1H, d, J=13Hz, $CH_2$-Pyrrol.); 3,82 (1H, d, J=18Hz, S—$CH_2$); 3,43 (1H, d, J=18Hz, S—$CH_2$); 3,28 (4H, m, Pyrrol.); 3,07 (2H, m,

—$CH_2$—$^+$N—);

2,04 (4H, m, Pyrrol.); 1,58 (2H, m, —$CH_2$—); 1,18 (2H, m, —$CH_2$—); 0,80 (3H, t, J=7Hz, $CH_3$).

Beispiel 11

7β-[(Z)-2-(2-Aminothiazol-4-yl)-2-methoxyiminoacetamido]-3-(1-butyl-1-pyrrolidinium)methyl-3-cephem-4-carboxylat

Die Herstellung erfolgt analog Beispiel 3 aus (Z)-2-(2-Aminothiazol-4-yl)-2-methoxyiminoessigsäure und 7-Amino-3-(1-butyl-1-pyrrolidinium)methyl-3-cephem-4-carboxylat

$^1$H-NMR (DMSO-$d_6$)

δ (ppm) = 9,58 (1H, d, J=9Hz, NH); 7.21 (1H, bs, $HN_2$); 6.71 (1H, s, Thiazol); 5.62 (1H, dd, J = 9Hz, J=5Hz, H-7-Lactam; 5.10 (1H, d, J=5Hz, H-6-Lactam; 5,05 1H, d, J=13Hz, $CH_2$-Pyrrol.); 3,86 (3H, s, $OCH_3$); 3,82 (2H, m, $CH_2$-Pyrrol, S—$CH_2$); 3,30 (5H, m, S—$CH_2$, Pyrrol); 3,13 (2H, m,

—$CH_2$—$^+$N—);

2.01 (4H, m, Pyrrol.); 1,70 (2H, m, —$CH_2$—); 1,27 (2H, m, —$CH_2$); 0,90 (3H, t, J=7Hz, $CH_3$).

Beispiel 12

3-[1-(3-Hydroxypropyl)-1-pyrrolidinium]methyl-7β-phenylacetamido-3-cephem-4-carboxylat

Die Herstellung erfolgt analog Beispiel 4 aus 3-Actoxymethyl-7β-phenylactamido-3-cephem-4-carbonsäure und N-(3-Hydroxypropyl)-pyrrolidin

$^1$H-NMR ($D_6$-DMSO)

δ (ppm) = 9,16 (1H, d, J=9Hz, NH); 7,30 (5H, arom.); 5.58 (1H, dd, J=9Hz, J=5Hz, H-7-Lactam); 5,08 (1H, d, J=5Hz, H-6-Lactam); 5,03 (1H, d, J=13Hz, $CH_2$-Pyrrol.); 3,88 (1H, d, J=13Hz, $CH_2$-Pyrrol.); 3,84 (1H, d, J=18Hz, S—$CH_2$); 2.90—3,60 (11H, m); 1,80—2,10 (6H, m).

Beispiel 13

7-Amino-3-[1-(3-hydroxypropyl)-1-pyrrolidinium]metyl-3-cephem-4-carboxylat

Die Herstellung erfolgt analog Beispiel 5 aus 3-[1-(3-Hydroxypropyl)-1-pyrrolidinium]methyl-3-cephem-4-carboxylat

$^1$H-NMR ($D_2O$)

δ (ppm) = 5,31 (1H, d, J=5Hz, H-7-Lactam); 5,11 (1H, d, J=5Hz, H-6-Lactam); 4,63 (1H, d, J=13Hz, $CH_2$-Pyrrol.); 3.96 (1H, d, J=13Hz, $CH_2$-Pyrrol.); 3,88 (1H, d, J=18Hz, S—$CH_2$); 3,40—3,60 (9H, m); 2,08 (4H, m, Pyrrol.); 1,92 (2H, m, —$CH_2$—).

Beispiel 14

7β-[(Z)-2-(2-Aminothiazol-4-yl)-2-methoxyiminoacetamido]3-[1-(3-hydroxypropyl)-1-pyrrolidinium]methyl-3-cephem-4-carboxylat

Die Herstellung erfolgt analog Beispiel 3 aus (Z)-2-(2-Aminothiazol-4-yl)-methoxyiminoessigsäure und 7-Amino-3-[1-(3-hydroxypropyl)1-pyrrolidinium]methyl-3-cephem-4-carboxylat

$^1$H-NMR (DMSO-$d_6$)

δ (ppm) = 9,59 (1H, d, J=9Hz, NH); 7,26 (2H, bs, $NH_2$); 6,75 (1H, s, Thiazol); 5,68 (1H, dd, J=9Hz, J=5Hz,

H-7-Lactam); 5,13 (1H, d, J=5Hz, H-6-Lactam); 5,05 (1H, d, J=13Hz, $CH_2$-Pyrrol.); 3,86 (3H, s, $OCH_3$); 3,84 (2H, m, $CH_2$-Pyrrol., S—$CH_2$); 3,20—3,60 (9H, m, S—$CH_2$,

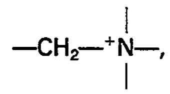

—$CH_2OH$'Pyrrol.); 1,88 (2H, m, —$CH_2$—).

Beispiel 15

3-{1-[2-(2-Hydroxyethoxy)ethyl]-1-pyrrolidinium}methyl-7β-phenylacetamido-3-cephem-4-carboxylat

Die Herstellung erfolgt analog Beispiel 4 aus 3-Acetoxymethyl-7β-phenylacetamido-3-cephem-4-carbonsäure und N-[2-(2-Hydroxyethoxy)ethyl]-pyrrolidin

$^1$H-NMR ($D_6$-DMSO)

δ (ppm) = 9,15 (1H, d, J=9Hz, NH); 7,31 (5H, m, arom.); 5,57 (1H, dd, J=9Hz, J=5Hz, H-7-Lactam); 5.15 (1H, d, J=13Hz, $CH_2$-Pyrrol.); 5.08 (1H, d, J=5Hz, H-6-Lactam); 3,99 (1H, d, J=13Hz, $CH_2$-Pyrrol.); 2,90—3,90 (14H, m); 2,08 (4H, m, Pyrrol.).

Beispiel 16

7-Amino-3-{1-[2-(2-hydroxyethoxy)ethyl]-1-pyrrolidinium}methyl-3-cephem-4-carboxylat

Die Herstellung erfolgt analog Beispiel 5 aus 3-{1-[2-(2-Hydroxyethoxy)ethyl]-1-pyrrolidinium}methyl-7β-phenylacetamido-3-cephem-4-carboxylat

$^1$H-NMR ($D_2O$)

δ (ppm) = 5,25 (1H, d, J=5Hz, H-7-Lactam); 5,07 (1H, d, J=5Hz, H-6-Lactam); 4,65 (1H, d, J=13Hz, $CH_2$-Pyrrol.); 4,04 (1H, d, J=13Hz, $CH_2$-Pyrrol.); 3,82 (1H, d, J=18Hz, S—$CH_2$); 3,30—3.80 (9H, m); 2,05 (4H, m, Pyrrol.).

Beispiel 17

7β-[(Z)-2-(2-Aminothiazol-4-yl)-2-methoxyiminoacetamido]-3-{1-[2-(2-hydroxyethoxy)ethyl]-1-pyrrolidinium}methyl-3-cephem-4-carboxylat

Die Herstellung erfolgt analog Beispiel 3 aus (Z)-2-(2-Aminothiazol-4-yl)-2-methoxyiminoessigsäure und 7-Amino-3-{1-[2-(2-hydroxyethoxy)ethyl]-1-pyrrolidinium}methyl-3-cephem-4-carboxylat

$^1$H-NMR (DMSO-$d_6$)

δ (ppm) = 9,60 (1H, d, J=9Hz, NH); 7,27 (2H, bs, $NH_2$); 6,74 (1H, s, Thiazol); 5,67 (1H, dd, J=9Hz, J=5Hz, H-7-Lactam; 5,16 (1H, d, J=13Hz, $CH_2$-Pyrrol.); 5,14 (1H, d, J=5Hz, H-6-Lactam); 3,98 (1H, d, J=13Hz, $CH_2$-Pyrrol.); 3,84 (3H, s, $OCH_3$); 3,80 (3H, m); 3.30—3,60 (11H, m); 2,05 (4H, m, Pyrrol.).

Beispiel 18

3-[1-(2-Hydroxy-2-phenylethyl)1-pyrrolidinium]methyl-7β-phenylacetamido-3-cephem-4-carboxylat

Die Herstellung erfolgt analog Beispiel 4 aus 3-Acetoxymethyl-7β-phenylacetamido-3-cephem-4-carbonsäure und DL—N-(2-Hydroxy-2-phenylethyl)-pyrrolidin. Man erhält eine Gemisch aus zwei Diastereoisomeren.

$^1$H-NMR ($D_6$-DMSO)

δ (ppm) = 9,14 (1H, d, J=9Hz, NH); 7.20—7.60 (10H, m, arom.); 5,58 (1H, m, H-7-Lactam); 5,33 (1H, m, CH—OH); 5.19 (1H, m, $CH_2$-Pyrrol.); 5,07 (1H, d, J=5Hz, H-6-Lactam; 4,36 u. 4,21 (1H, d, J=13Hz, $CH_2$-Pyrrol.); 3,10—390 (10H, m); 2,10 (4H, m, Pyrrol.).

Beispiel 19

7-Amino-3-[1-(2-hydroxy-2-phenylethyl)-1-pyrrolidinium]methyl-3-cephem-4-carboxylat

Die Herstellung erfolgt analog Beispiel 5 aus 3-[1-(2-Hydroxy-2-phenylethyl)-1-pyrrolidinium]methyl-3-cephem-4-carboxylat (Gemisch aus zwei Diastereoisomeren).

$^1$H-NMR ($D_2O$)

δ (ppm) = 7,40 (5H, bs, arom.); 5,30 (2H, m, H-7-Lactam, CH—OH); 5,14 (1H, m H-6-Lactam); 4,82 (1H, m, $Ch_2$-Pyrrol.); 4.55 u. 4,37 (1H, d, J=13Hz, $CH_2$-Pyrrol.); 3,30—4,00 (8H, m); 2,16 (4H, bs, Pyrrol.).

Beispiel 20

7β-[(Z)-2-(2-Aminothiazol-4-yl)-2-methoxyiminoacetamido]3-[1-(2-hydroxy-2-phenylethyl)-1-pyrrolidinium]methyl-3-cephem-4-carboxylat

Die Herstellung erfolgt analog Beispiel 3 aus (Z)-2-(2-Aminothiazol-4-yl)-2-methoxyiminoessigsäure und 7-Amino-3-[1-(2-hydroxy-2-phenylethyl)-1-pyrrolidinium]methyl-3-cephem-4-carboxylat (Gemisch aus zwei Diastereoisomeren).

$^1$H-NMR (DMSO-$d_6$)

δ (ppm) = 9,58 (1H, d, J=9Hz, NH); 7,30—7,50 5H, m, arom.); 7,22 (2H, bs, $NH_2$); 6,72 und 6,73 (1H, s, Thiazol); 5,63 (1H, m, H-7-Lactam); 5,08—5,30 (3H, CH—OH, $CH_2$-Pyrrol., H-6-Lactam); 4,29 u, 4,13 (1H, d, J=13Hz, $CH_2$-Pyrrol.); 3,82 (3H, s, $OCH_3$); 3,20—3,80 (8H, m); 2,05 (4H, m, Pyrrol.).

**Patentansprüche**

1. Cephalosporine der allgemeinen Formel I und deren pharmazeutisch verträgliche Salze

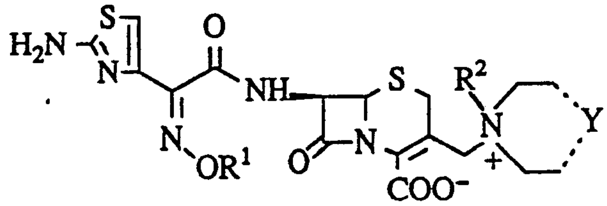

(I)

in der

$R^1$ einen $C_1$—$C_3$-Alkylrest darstellt und

$R^2$ für Propyl, Isopropyl, Cyclopropyl, Chlorethyl, Methoxymethyl, Methoxyethyl, Vinyl, Carboxyethyl oder Aminoethyl steht, und

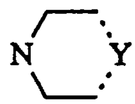 für einen Rest der Formel

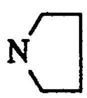 , 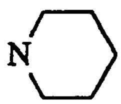 ; 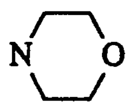 , 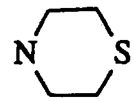 , 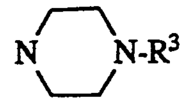

steht, worin $R^3$ die oben für $R^2$ genannte Bedeutung hat.

2. Cephalosporine nach Anspruch 1, in denen

$R^1$ einen Methylrest darstellt.

3. Verbindungen aus der Gruppe bestehend aus

7β-[(Z)-2-(2-Aminothiazol-4-yl)-2-methoxyiminoacetamido]-3-(1-butyl-1-pyrrolidinium)methyl-3-cephem-4-carboxylat

7β-[(Z)-2-(2-Aminothiazol-4-yl)-2-methoxyiminoacetamido]-3-[1-(3-hydroxypropyl)-1-pyrrolidinium]methyl-3-cephem-4-carboxylat

7β-[(Z)-2-(2-Aminothiazol-4-yl)-2-methoxyiminoacetamido]-3-{1-(2-(2-hydroxyethoxy)ethyl]1-pyrrolidinium}methyl-3-cephem-4-carboxylat

7β-[(Z)-2-(2-Aminothiazol-4-yl)-2-methoxyiminoacetamido]3-[1-(2-hydroxy-2-phenylethyl)-1-pyrrolidinium]methyl-3-cephem-4-carboxylat

4. Verbindungen der allgemeinen Formel III

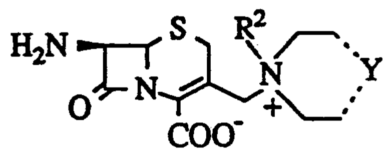

(III)

in der

$R^2$ für Propyl, Isopropyl, Cyclopropyl, Chlorethyl, Methoxymethyl, Methoxyethyl, Vinyl, Carboxyethyl oder Aminoethyl, 1-Butyl, 3-hydroxypropyl, 2-Hydroxyethoxyethyl oder 2-Hydroxy-2-phenylethyl steht, und

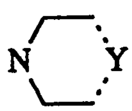 für einem Rest der Formel

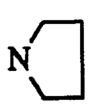 , 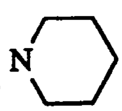 ; 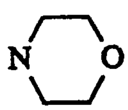 , 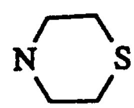 , 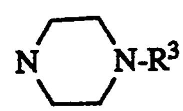

steht, worin $R^3$ die oben für $R^2$ genannte Bedeutung hat.

5. Verfahren zur Herstellung von Cephalosporinen der allgemeinen Formel I

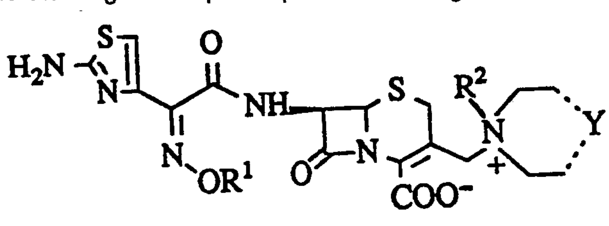

(I)

in der

$R^1$ einen $C_1$—$C_3$-Alkyrest darstellt und

$R^2$ für Propyl, Isopropyl, Cyclopropyl, Chlorethyl, Methoxymethyl, Methoxyethyl, Vinyl, Carboxyethyl oder Aminoethyl, steht, und

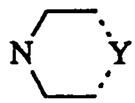 für einem Rest der Formel

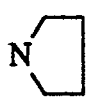 , 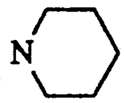 ; 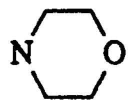 , 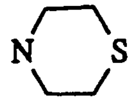 , 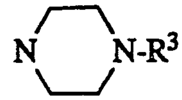

steht, worin $R^3$ die oben für $R^2$ genannte Bedeutung hat, dadurch gekennzeichnet, daß Verbindungen der allgemeinen Formel II

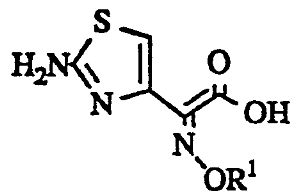

(II)

worin

$R^1$ die oben genannte Bedeutung hat und in denen die Amingruppe geschützt oder ungeschützt vorliegen kann, nach Aktivierung der Carboxylgruppe durch Überführung in ein gemischtes Anhydrid, nach Überführung in das Säurehalogenid oder nach Überführung in einen aktivierten Ester mit Verbindungen der allgemeinen Formel III,

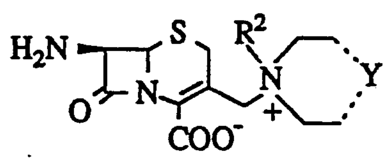

(III)

worin $R^2$ und

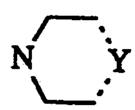

die oben genannte Bedeutung besitzen, zur Umsetzung gebracht werden, dann gegebenenfalls Schutzgruppen abgespalten werden und die gewünschten Salze oder aus Salzen die freie Säure hergestellt werden.

6. Arzneimittel, enthaltend mindestens eine Verbindung nach einem der Ansprüche 1, 2 und 3.

7. Verbindungen gemäß einem der Ansprüche 1 und 2 zur Anwendung in einem Verfahren zur therapeutischen Behandlung des menschlichen oder tierischen Körpers.

8. Verwendung der Verbindungen gemäß einem der Ansprüche 1, 2 und 3 zur Herstellung von Arzneimitteln.

**Revendications**

1. Céphalosporines de formule générale I:

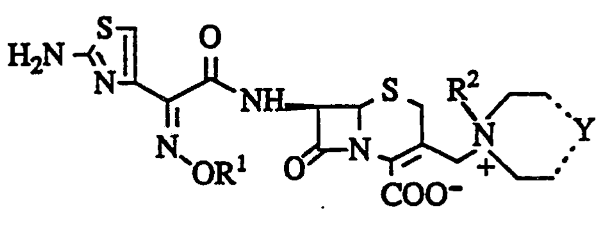

(I)

dans laquelle

$R^1$ représente un reste alkyle en $C_1$ à $C_3$, et

$R^2$ représente un groupe propyle, isopropyle, cyclpropyle, chloroéthyle, méthoxyméthyle, méthoxyéthyle, vinyle, carboxyéthyle et aminoéthyle, et

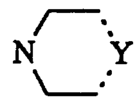 represénte un reste de formule

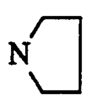 , 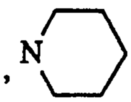 ; 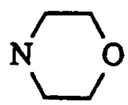 , 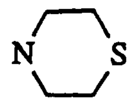 , 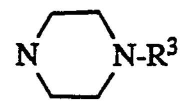

(où $R^3$ a le sens indiqué ci-dessus pour $R^2$)], et leurs sels pharmaceutiquement tolérables.

2. Céphalosporines selon la revendication 1, dans lesquelles:
$R^1$ représente un reste méthyle.

3. Composés choisis dans l'ensemble consistant en:
un 7β-[(Z)-2-(2-aminothiazol-4-yl)-2-méthoxyiminoacétamido]-3-(1-butyl-1-pyrrolidinium)méthyl-3-céphem-4-carboxylate,
un 7β-[(Z)-2-(2-aminothiazol-4-yl)-2-méthoxyiminoacétamido]-3-[1-(3-hydroxypropyl)-1-pyrrolidinium]méthyl-3-céphem-4-carboxylate,
un 7β-[(Z)-2-(2-aminothiazol-4-yl)-2-méthoxyiminoacétamido]-3-{1-[2-2-hydroxyéthoxy)éthyl]1-pyrrolidinium méthyl-3-céphem-4-carboxylate,
un 7β-[(Z)-2-(2-aminothiazol-4-yl)-2-méthoxyiminoacétamido]-3-[1-(2-hydroxy-2-phényléthyl)-1-pyrrolidinium]méthyl-3-céphem-4-carboxylate.

4. Composés de formule générale III:

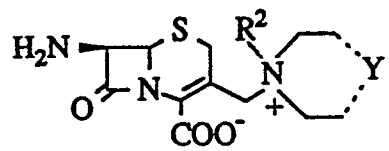 (III)

dans laquelle
$R^2$ représente un groupe propyle, isopropyle, cyclopropyle, chloréthyle, méthoxyméthyle, méthoxyéthyle, vinyle, carboxyéthyle ou aminoéthyle, 1-butyle, 3-hydroxypropyle, 2-hydroxyéthoxyéthyle ou 2-hydroxy-2-phényléthyle, et

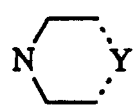 représente un reste de formule

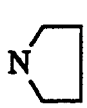 , 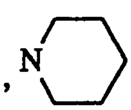 ; 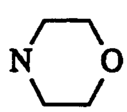 , 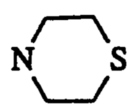 , 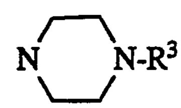

dans lesquels
$R^1$ a le sens indiqué ci-dessus pour $R^2$.

5. Procédé pour préparer des céphalosporines de formule générale I:

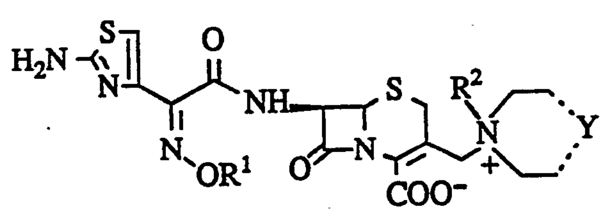 (I)

dans laquelle
$R^1$ représente un reste alkyle en $C_1$ à $C_3$, et
$R^2$ représente un groupe propyle, isopropyle, cyclopropyle, chloréthyle, méthoxyméthyle, méthoxyéthyle, vinyle, carboxyéthyle ou aminoéthyle, et

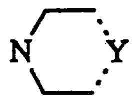 represénte un reste de formule

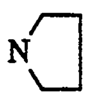, 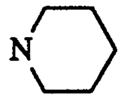; 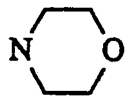, 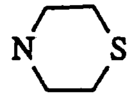, 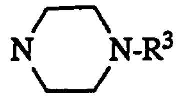

dans lesquels $R^3$ a le sens indiqué ci-dessus pour $R^2$, procédé caractérisé en ce qu'on fait réagir des composés de formule générale II:

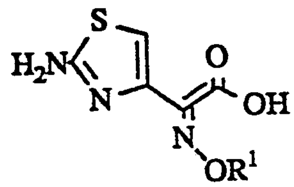

(II)

(dans laquelle

$R^2$ a le sens précité, et

dans lesquels le groupe amine peut être protégé ou non protégé), après activation du groupe carboxyle par sa transformation en un anhydride mixte, après transformation en halogénure d'acide ou après transformation et un ester activé, avec des composés de formule générale III:

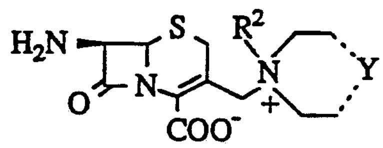

(III)

dans laquelle $R^2$ et

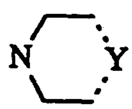

ont le sens précité,

puis l'on scinde éventuellement les groupes protecteurs et l'on prépare les sels voulus ou bien, à partir des sels, l'acide libre.

6. Médicament, contenant au moins un composé selon l'une des revendications 1, 2 et 3.

7. Composés selon l'une des revendications 1 et 2 à utiliser dans un procédé de traitement thérapeutique du corps humain ou animal.

8. Utilisation des composés selon l'une des revendications 1, 2 et 3 pour préparer des médicaments.

**Claims**

1. Cephalosporins of the general formula I and their pharmaceutically tolerable salts

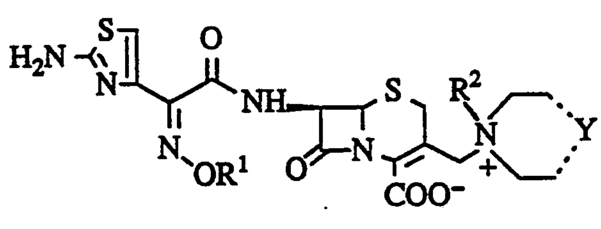

(I)

in which

$R^1$ represents a $C_1$—$C_3$-alkyl radical and

$R^2$ represents propyl, isopropyl, cyclopropyl, chloroethyl, methoxymethyl, methoxyethyl, vinyl, carboxyethyl or aminoethyl, and

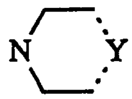 represents a radical of the formula

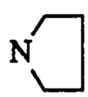 , 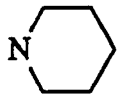 ; 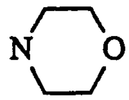 , 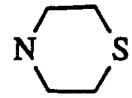 , 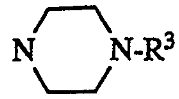

in which $R^3$ has the meaning mentioned above for $R^2$.

2. Cephalosporins according to Claim 1, in which $R^1$ represents a methyl radical.

3. Compounds from the group comprising

7β-[(Z)-2-(2-aminothiazol-4-yl)-2-methoxyiminoacetamido]-3-(1-butyl-1-pyrrolidinium)methyl-3-cephem-4-carboxylate

7β-[(Z)-2-(2-aminothiazol-4-yl)-2-methoxyiminoacetamido]-3-[1-(3-hydroxypropyl)-1-pyrrolidinium]methyl-3-cephem-4-carboxylate

7β-[(Z)-2-(2-aminothiazol-4-yl)-2-methoxyiminoacetamido]-3-{1-[2-(2-hydroxyethoxy)ethyl]1-pyrrolidinium}methyl-3-cephem-4-carboxylate

7β-[(Z)-2-(2-aminothiazol-4-yl)-2-methoxyiminoacetamido]3-[1-(2-hydroxy-2-phenylethyl)-1-pyrrolidinium]methyl-3-cephem-4-carboxylate.

4. Compounds of the general formula III

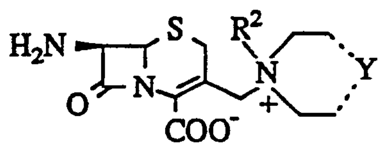 (III)

in which

$R^2$ represents propyl, isopropyl, cyclopropyl, chloroethyl, methoxymethyl, methoxyethyl, vinyl, carboxyethyl or aminoethyl, 1-butyl, 3-hydroxypropyl, 2-hydroxyethoxyethyl or 2-hydroxy-2-phenylethyl, and

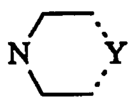 represents a radical of the formula

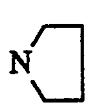 , 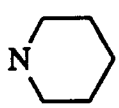 ; 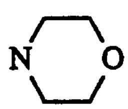 , 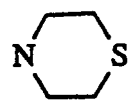 , 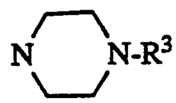

in which $R^3$ has the meaning mentioned above for $R^2$.

5. Process for the preparation of cephalosporins of the general formula I

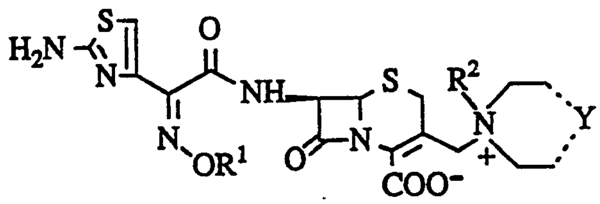 (I)

in which

$R^1$ represents a $C_1$—$C_3$-alkyl radical and

$R^2$ represents propyl, isopropyl, cyclopropyl, chloroethyl, methoxymethyl, methoxyethyl, vinyl, carboxyethyl or aminoethyl, and

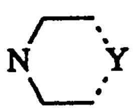 represents a radical of the formula

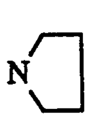 , 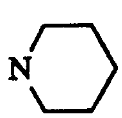 ; 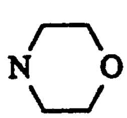 , 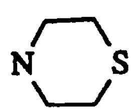 , 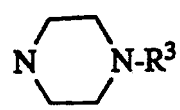

in which $R^3$ has the meaning mentioned above for $R^2$, characterized in that compounds of the general formula II

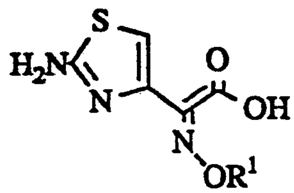

(II)

in which

$R^1$ has the abovementioned meaning and in which the amine group can be protected or unprotected, are reacted, after activation of the carboxyl group by conversion into a mixed anhydride, after conversion into the acid halide or after conversion into an activated ester, with compounds of the general formula III,

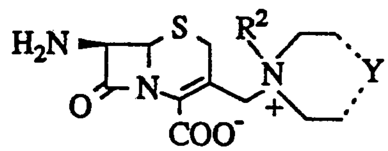

(III)

in which $R^2$ and

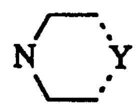

have the abovementioned meaning,
and then, if appropriate, protective groups are removed and the desired salts, or, from salts, the free acids are prepared.

6. Medicaments containing at least one compound according to one of Claims 1, 2 and 3.

7. Compounds according to one of Claims 1 and 2 for use in a method for the therapeutic treatment of the human or animal body.

8. Use of the compounds according to one of Claims 1, 2 and 3 for the production of medicaments.